# EUROPEAN PATENT SPECIFICATION

(11) **EP 3 389 435 B1**
(45) Date of publication and mention of the grant of the patent: **04.01.2023**
(21) Application number: 16806114.1
(22) Date of filing: 06.12.2016
(51) Int. Cl.: A45D 34/00, A45D 34/04, A61H 15/02, A61N 1/04

(54) **DEVICE FOR TREATING HUMAN KERATINOUS MATERIAL, ESPECIALLY WITH THE AID OF AN ELECTRIC CURRENT**
VORRICHTUNG ZUR BEHANDLUNG VON MENSCHLICHEM KERATINMATERIAL, INSBESONDERE MIT HILFE EINES ELEKTRISCHEN STROMS
DISPOSITIF POUR TRAITER UNE MATIÈRE HUMAINE KÉRATINEUSE, NOTAMMENT AVEC L'AIDE D'UN COURANT ÉLECTRIQUE

(30) Priority: 17.12.2015 FR 1562636
(43) Date of publication of application: 24.10.2018
(73) Proprietor: L'OREAL, 75008 Paris (FR); SEB S.A., 69130 Ecully (FR)
(72) Inventor: PLANARD-LUONG, Thi Hong Lien, 94152 Chevilly Larue (FR)
(74) Representative: Nony
(86) International application number: PCT/EP2016/079923
(87) International publication number: WO 2017/102452

(56) References cited:
- WO-A1-2014/180555
- WO-A1-2015/091044
- US-A- 5 131 384

## Description

The present invention relates to devices intended to carry out a treatment, in particular cosmetic or dermatological, of keratinous material, in particular of the skin, of the scalp, or of the hair. By "cosmetic or dermatological composition" is meant any composition as defined in Directive 93/35/CEE of the Council of 14 June 1993.

The invention relates in particular to devices for the treatment, in particular cosmetic or dermatological, of keratinous material with the aid of an electric current.

It is known that the application of an electric current to the skin may facilitate the penetration of an active agent. It is thus known to treat human keratinous material with the aid of iontophoresis devices. Iontophoresis allows the diffusion of active agents through the skin by virtue of an electrical stimulation in a non-invasive manner. The current administered may be adjustable in terms of intensity and polarity (anodic or cathodic current). The transcutaneous diffusion of the molecules via iontophoresis is based on two principles, namely electrorepulsion and electro-osmosis.

Electrorepulsion is the migration of an ionized molecule by repulsion of charge of the same sign. Thus, a positively charged substance will diffuse through the skin at the level of the anode (+).

Electro-osmosis is the migration of a molecule, even non-ionized, by entrainment related to the stream of water from the anode toward the cathode during iontophoresis. Under the effect of a current, dissolved substances are entrained by the water or a solvent during migration.

Patent US 5 090 402 relates to an electric current applicator also exhibiting a massaging function, comprising a plurality of balls, all the balls being immersed in the composition reservoir which extends under a support of the balls.

The subject of patent US 5 131 384 is a massaging applicator comprising a plurality of balls, including a central ball supplied directly with product. This massaging applicator is devoid of a system for applying an electric current.

In patent application US 2005/0107832 the applicator comprises a matrix of electrodes.

Document WO2015/091044 discloses a device for applying a cosmetic composition to the skin and for exposing the skin to an electric treatment current in a zone of application of the composition.

A need exists to further enhance devices which facilitate the penetration of active agents through the skin so as to increase the efficacy of iontophoresis.

A need exists in particular to benefit from a device for the treatment, in particular cosmetic or dermatological, of keratinous material, in particular with the aid of an electric current, which exhibits good efficacy and may be used with comfort and in complete safety.

The above needs are met by providing a device according to claim 1.

Thus, the first position may be a position of the application member when at rest in its housing and the second position is the position of the application member which moves in its housing under the pressure of the composition.

By "human keratinous material" is meant mainly the skin, in particular of the body or of the face, or else the scalp, the nails or the hair.

The presence of the intermediate chamber allows intermediate storage of a small quantity of composition, thereby possibly allowing the dispensing of the composition onto the keratinous material to be smoothed even if the dispensing thereof by the dispensing member from the reservoir occurs jerkily, i.e. in fits and starts, this possibly in particular being the case when the dispensing member is actuated manually, for example by means of a dispensing button, or automatically, for example by means of a jerky motor. The dispensing member may thus be configured to dispense the composition jerkily.

The dispensing member may be actuated manually. As a variant, the dispensing member is actuated automatically, for example by means of an electric motor. The electric motor may be different from a stepper motor, thereby making it possible to benefit from a less expensive device.

The dispensing member may comprise at least one motor, in particular at least one geared motor without servocontrol, for example a DC motor. Such a device may allow the motor to be powered for a given time interval and not continuously. The dispensing member may in particular be devoid of a worm wheel, or of a stepper motor, or of a reduction gear motor servo-controlled in terms of revolution count.

The maximum volume of the intermediate chamber made between the bottom of the housing and the application member disposed in said housing may be between 5 and 100 microliters (µL), better between 10 and 50 µL, being for example of the order of 20 µL.

The device may be configured to allow a flowrate of composition dispensed onto the keratinous material of between 0.5 and 20 µL/s, better between 1 µL/s and 10 µL/s, being for example of the order of 2 to 4 µL/s. The device according to the invention thus advantageously makes it possible to dispense the composition with a very low flowrate, and this may be advantageous in the case of an iontophoresis treatment.

The presence of the intermediate chamber makes it possible to improve the control of the flowrate at the exit of the device, whatever the nature of the dispensing member used. Preferably, the dispensing of the composition at the exit of the device is thus done in a continuous manner and smoothly, even if the dispensing by the dispensing member may occur jerkily. The regularity of the composition dispensing flowrate is important in an iontophoresis treatment since it makes it possible to guarantee the efficacy and the stability of the micro-current applied and therefore the comfort of the user during the treatment.

The use of the device according to the invention is particularly advantageous in the case of a composition whose viscosity varies over time during the use of the device. Indeed, the presence of the intermediate chamber makes it possible to compensate for the viscosity variation of the composition. The viscosity variation may result from long-duration storage in the reservoir over the duration of use of the quantity of composition contained in the reservoir.

The housing is supplied with composition from the reservoir through a supply orifice. The supply orifice may be disposed in the bottom of the housing, or as a variant be disposed elsewhere than in the bottom of the housing.

A height *h* of the intermediate storage chamber extends between the first position of the application member and its second position.

The housing may have a shape configured so as to retain the application member in the latter. Thus, the shape of the housing may be chosen so as to prevent the application member from escaping from the latter.

The housing may have an opening of smaller cross-section than a larger transverse cross-section of the application member. The opening of the housing allows the application of the composition to the keratinous material by means of the application member which protrudes from the housing therethrough.

The housing has a transverse cross-sectional shape that is larger than a semicircle, the application member being of circular shape in transverse cross-section. The transverse cross-section of the housing has for example the shape of a circular arc corresponding to 51% to70% of the perimeter of a circle, better 52% to 65% of perimeter of a corresponding circle, better still 53% to 60% of the perimeter of a corresponding circle. By "transverse cross-section" is meant a cross-section taken parallel to a longitudinal axis of the device, and if appropriate perpendicularly to a longitudinal axis of the application member in the case where the latter has a longitudinal axis, for example in the case where the latter is cylindrical.

The housing has for example a partially spherical shape, in particular slightly greater than a hemisphere, in the case where the application member has a ball shape. In this case, the contours of the application member and of its housing may be concentric. The radii of curvature of the application member and of its housing may be equal to within 20%, better to within 10%, or indeed to within 5%. The disparity between these radii of curvature enables the intermediate chamber to be made.

As a variant, in particular in the case where the application member has a cylinder shape, being for example a roller, the housing may have an envelope of slightly larger shape than a half-cylinder.

As a variant, the application member may be retained in the housing by means of a cover. The housing may in this case have an envelope having a shape with smaller transverse cross-section or equal to a semicircle.

In a further variant, the bottom of the housing is slightly flattened.

The device may comprise a plurality of housings, in particular one for each application member.

The end fitting may comprise a distributor comprising at least one storage cavity for the composition and supply orifices to conduct the composition from the storage cavity or cavities to the application members.

A part at least of the application members might not lie above the cavity or cavities. The cavity or cavities of the distributor do not extend under the entirety of the application members. The plurality of the application members covers the cavity or cavities and protrudes around the cavity or cavities when the distributor is observed along a longitudinal axis of the end fitting.

The distributor may be configured so that the flowrate of the composition arriving at the application members is identical to within 20%, better to within 15%, or indeed to within 10%, for at least two application members, or indeed for each application member. The flowrate of the composition arriving at the application members is the flowrate of the composition exiting the supply orifice corresponding to the application member in question, to supply the housing of said application member. Two flow rates D₁ and D₂ are compared in the following way. D₁ being the smaller flowrate of the two and D₂ the larger flowrate of the two, the following ratio is calculated: (D₂-D₁)/D₂.

### Electrically conducting distributor

The distributor may be electrically conducting.

The material or materials of the distributor may be chosen from the following list, which is non-limiting: acetal, polyester, ABS, polyamide, polycarbonate, PP, PE, silicone, butyl, nitrile, viton, PBT, and combination of these materials. It may in particular be a filled plastic, for example polycarbonate preferentially.

The material of the distributor may be rendered electrically conducting by inserting a filler into the polymer matrix, this filler being able to be chosen from the following list, which is not limiting: metal, graphite, fiber or carbon powder.

In one embodiment, the distributor is formed from at least one thermoplastic, which comprises an electrically conducting filler, for example a carbon filler or a metallic filler. A carbon filler is preferred so as to ensure that the distributor is fairly lightweight.

In one embodiment, the application members are not electrically conducting.

The distributor may be electrically connected to an electrical supply system by a metallic lug. The latter may make it possible to ensure the transfer of electric current from the electrical supply system to the distributor. The distributor and the composition may be the only components in the end fitting that are electrically conducting. In particular, the cap, the cover and/or the application members might not be electrically conducting. The only element in contact with the skin and which is electrically conducting may be the composition.

### Application member

The device may comprise several application members, in particular between two and eight, better between three and six, in particular in the form of balls. The multiplicity of the application members facilitates good distribution of the composition, in a continuous manner, and improves the ionization of the composition during the treatment, if appropriate. The multiplicity of the application members also makes it possible to improve the massaging effect in the course of the treatment.

At least one application member may be a ball, and better all the application members are balls.

As a variant, the application members may exhibit any profile, in particular cylindrical, being for example in the form of rollers, or non-cylindrical, for example an ovoid or discoid shape.

At least some of the application members may be organized as a polygon, in particular as a triangle, and better all the application members are organized as a polygon, in particular as a triangle. Such a disposition facilitates the application and the treatment of zones which are difficult to access, for example the wings of the nose.

This configuration of the application members also facilitates good distribution of the composition over the whole of the application surface. Better homogeneity of the treatment is thus obtained. Safety and comfort which are related to good distribution of the electric current on the application surface are also improved, if appropriate. The disposition of the application members and the relatively high number thereof allow simultaneous application over a fairly large surface area.

The application members may be made of plastic or metal.

The application members are preferably neither electrically conducting, nor linked to the electrical supply circuit by metal conductors.

The outer surface of an application member may be totally inert from a chemical point of view in relation to the composition applied and the keratinous material. The outer surface may be covered with a varnish. The outer surface may be polished. The outer surface may comprise a biocide material, if appropriate.

Moreover, the outer surface of an application member may be disposed in proximity to the composition reservoir, thereby making it possible to avoid the presence of specific ducts for transporting the composition from the reservoir to this outer surface.

The application members may be spherical or cylindrical, roller-shaped for example, or have some other shape, especially ovoid.

Advantageously, at least one application member, better each application member, turns about at least one rotation axis. In an exemplary implementation of the invention, the outer surface of the application member exhibits a symmetry substantially of revolution about an axis of symmetry, the outer surface being able to be rotary around this axis of symmetry. As a variant, the application member may be rotary about a rotation axis distinct from this axis of symmetry.

At least one application member may comprise a core to which an outer wall is attached. This may be the case for all the application members. This core may comprise a surface provided with reliefs and the outer wall may be able to deform during application so as to come into contact with the reliefs. The latter makes it possible to produce a massaging effect when the application member is in contact with the surface to be treated, and this may inter alia help the penetration of the composition into the skin and facilitate its action.

The outer wall may, as a variant, be rigid.

In an exemplary implementation of the invention, the outer wall comprises reliefs, which may comprise bumps or ribs.

Alternatively, at least one application member may comprise removable elements which give the application member a relief. It is thus possible to change these elements with a view to modifying the dimensions of the application members, their surface properties, or else their roughness.

Advantageously, at least one application member is mounted in a removable manner on the device. All the application members may be mounted in a removable manner on the device.

The distributor may comprise a first face on which are made housings for the application members, in particular a housing for each of the application members. The first face may be plane, concave or convex. It is for example slightly domed.

The supply orifices are configured to conduct the composition from the cavity or cavities to the housings of the application members. The supply orifices may emerge in the housings elsewhere than in the bottom of the housings. This may make it possible to reduce the thickness of the distributor, so that the latter is more slender and less bulky.

Preferably, as mentioned hereinabove, a part at least of the housings of the application members does not lie above the cavity or cavities, in particular when the distributor is observed perpendicularly to the first face of the distributor. The cavity or cavities of the distributor do not then extend under the entirety of the housings of the application members. The plurality of housings covers the cavity or cavities and protrudes around the cavity or cavities when the distributor is observed perpendicularly to the first and second faces of the distributor.

The distributor comprises a second face opposite to the first, on which is made said at least one storage cavity for the composition. The cavity or cavities may be hollowed out with respect to the second face, which may be plane, concave or convex. The cavities may take the form of channels made in the distributor.

The distributor may comprise peripheral cavities, in particular three peripheral cavities, each of the peripheral cavities being able to enable the composition to be conveyed to several application members.

At least one peripheral cavity may be configured to convey the composition to two application members, for example two adjacent application members. In particular, each of the peripheral cavities may be configured to convey the composition to two application members, for example two adjacent application members. Thus, the application members may be linked pairwise by a corresponding cavity, which may be channel-shaped.

The supply orifices of the housings of the application members may emerge for this purpose in these peripheral cavities. Thus, the path followed by the composition may be minimized, thereby making it possible to reduce the volume of the cavity or cavities and thus the dead volume of composition present outside the reservoir and not yet dispensed.

The length of the path traversed by the composition in order to arrive at a given housing may be substantially equal to the length of the path traversed by the composition in order to arrive at another housing, to within 30%, better to within 20%, or 10%, better still to within 5%. By "length of the path" is meant the shortest path made in the distributor between the point of arrival of the composition in the distributor and the point of arrival in the corresponding housing, when the device is in use.

At least two supply orifices may be of different transverse cross-sections, so as to compensate at least in part for a difference in the length of the path traversed by the composition in order to arrive at the corresponding housings.

It is thus possible to facilitate evenness of dispensing of the composition to the application members, and obtain a balanced supply flowrate of composition for each of the application members. "Balanced flowrate" is understood to mean that each of the application members receives a flowrate of composition which differs by less than 20% from the flowrate received by another application member, better by less than 10%, better still by less than 5%.

The shape of the distributor facilitates good distribution of the composition to the application members, in spite of the high number thereof and of their different distance from the axis and from the exit orifice of the reservoir. It is thus possible to balance the dispensing of the composition on each application member while minimizing the quantity of composition stored in the distributor. The volume of the cavity or cavities may thus be reduced to the minimum.

The distributor may comprise at least one central cavity, in particular a single central cavity. This central cavity may be intended to be supplied directly with composition from the reservoir, in particular via the diffuser which will be described further on.

The peripheral cavities may be disposed as fins around the central cavity. The peripheral cavities may be supplied through v-shaped paths from the central cavity. The peripheral cavities may thus communicate with the central cavity through passages made at the periphery of the central cavity.

As a variant, the device is devoid of any central cavity, the peripheral cavities being mutually independent, and not being able to communicate with one another in the distributor. In this case, the peripheral cavities may be supplied with composition directly from the reservoir, in particular via the diffuser which will be described hereinafter.

At least one peripheral cavity, or indeed each of the peripheral cavities, may have a rectilinear channel shape, a supply orifice emerging at each of the ends of this channel. When they are three in number, these peripheral cavities may be disposed on each of the sides of a triangle starting from one of the vertices of said triangle, for example on half-sides of the triangle.

Each of the peripheral cavities may be supplied from the diffuser which will be described further on, the latter comprising a star-shaped diffuser cavity with three branches, each branch of the star supplying one of the peripheral cavities of the distributor in its middle or at one of its ends. The headloss undergone by the composition from its entry into the diffuser and up to the application members is preferably the same for each application member, at the very least differs by less than 20%, better less than 10%, or indeed less than 5%. Thus, one obtains an equivalent path traversed by the composition for the supply of each of the application members.

### Diffuser

The device may comprise a diffuser directly in contact with the distributor, making it possible to feed the composition into the cavity or cavities of the distributor.

The distributor and the diffuser may each have a face in contact with the other of the distributor or of the diffuser. These faces may be plane. The surfaces of these faces may be tailored in such a way that there is in principle no composition that flows between the surfaces in contact. However, it is not possible to exclude the presence of a film of composition between these surfaces, without the clearance which accommodates this film constituting a cavity within the meaning of the invention.

The diffuser may comprise at least one metallic notch, for example three metallic notches making it possible to receive a metallic lug for electrical conduction, as explained further on. A first leaktight seal may be inserted between the diffuser and the distributor, in particular around the cavity or cavities of the latter. A second leaktight seal may also be provided under the diffuser, being held on the latter by a seal holding piece.

As mentioned hereinabove, the device may comprise a cover configured so as to retain the application member or members in the housings. The cover may comprise openings through which the application members protrude to the outside to allow the application of the composition. In one embodiment, the cover is not electrically conducting.

### Electrical supply system

The device comprises an electrical supply system for exposing the keratinous material to a treatment electric current in a zone of application of the composition.

The expression "electrical supply system" is understood to mean an electrical assembly that is able to induce a potential difference between one or more electrodes and a counter electrode. If the end fitting is placed on the person's face and if the counter-electrode is held in a hand, the potential difference is established between the person's face and their hand.

### Electrodes

The electrical supply system may include an electrode located remotely from the keratinous material and making contact with the composition, and a counter electrode, preferably coming into contact with the keratinous material in a zone that is not exposed to the composition.

According to the invention, by "electrode" is meant a positively charged (anode) or negatively charged (cathode) electrode. This electrode may be disposed in the application tip, so as to ensure the passage of the electric current into the composition. The electrode is then disposed inside the end fitting. In this case, it does not come directly into contact with the keratinous material, but with the composition itself. The composition may be the only conducting substance in contact with the skin during the use of the device. The electrode may not be in contact with the keratinous material, in particular the skin, the end fitting not comprising any electrically conducting material in contact with the skin.

Throughout the text, the term "electrode" signifies a single isolated electrode. An electrode may take for example the form of a ball, stud, tab. The device may comprise a single or several electrodes.

By "counter-electrode" is meant an electrode carried to a higher potential than the other electrode (cathode) or lower potential than the other electrode (anode). The sign of the polarity of the counter-electrode is opposite to that of the electrode. In general, said counter electrode is disposed on the body of the device or on a handpiece. The counter-electrode is intended to come into contact with a bodily zone of the person undergoing the care. For example, it may be grasped between the person's fingers or between their palm and fingers. In one configuration, the counter-electrode is disposed on the end fitting. In this eventuality, it is separated from the electrode by an insulating space and is distanced as far as possible from the electrode so as to avoid any leakage current.

The electrode may be housed inside the end fitting, the electrode being in particular distanced from the outer wall of the application member by a distance of between 0.2 mm and 5 mm. This distance represents the gap between the electrode and the outer surface of the application member. This distance is the shortest measurable distance between the electrode and the outer surface of the application member. It is measured between any point of the electrode and of the outer surface of the application member, provided that the measured distance is the shortest distance.

Advantageously, the electrical supply system includes a current generator able to control the magnitude of the treatment current flowing between the electrode and skin, thereby allowing the voltage U between the electrode and the counter electrode to be controlled. The voltage U generated depends on the impedance of the "skin + composition" system. The voltage U is limited to a maximum value Uₘₐₓ deliverable by the current generator, for safety reasons (about 100V for example). This voltage Uₘₐₓ may be less than 150V.

The electrode may be plane, for example in the form of a plane disc or of a polygon. The electrode may be hollow, being formed for example by stamping or bending an electrically conducting metal sheet. The electrode may be porous.

### Electrical parameters

The electrical energy source may comprise any cell or any accumulator. The voltage between the electrodes is for example between 1.2 V and 24 V, preferably between 1.2 and 3.3 V. If appropriate, the passage of the current may create pointlike heating.

At equivalent current density, the device may in particular deliver a current density, at the level of the skin, of preferably less than or equal to 0.500 mA/cm², for example between 0.01 mA/cm² and 0.500 mA/cm², for example between 0.1 mA/cm² and 0.3 mA/cm².

### Control system and warning facility

The device may furthermore comprise a control system sensitive to the impedance of the skin. The control system may be configured to perform a measurement giving information on the impedance of the skin. By "measurement" is meant that the measured magnitude is compared with a threshold value, for example whether or not the measured magnitude has exceeded the threshold value, or whether or not the measured magnitude lies in a given interval.

The device may furthermore comprise a warning facility providing the user with a signal in response to information originating from the control system sensitive to the impedance of the skin. The warning facility may thus make it possible to forewarn the user that the measured impedance has exceeded a threshold value.

The signal provided may in particular relate to the possible necessity to modify the current density between the electrodes and/or the quantity of composition to be dispensed. This modification may be performed automatically by a slaving system of the device, or else manually by the user. The signal may for example invite the user to dispense an additional dose of composition. The signal may as a variant or additionally invite the user to increase the current density.

The threshold value or values of the warning facility may be preprogrammed in the device, or else be fixed by the user, or else depend on the composition reservoir inserted into the device, if the reservoir is recognized by the device and if a corresponding threshold is automatically determined as a function of an identifier of the reservoir.

If there is insufficient composition between the electrode and the skin, the impedance is very high. The voltage U may become too high.

If the quantity of cosmetic composition between the electrode and the skin increases, the voltage U may decrease on account of a decrease in the impedance at this location.

The device may comprise a member for adjusting the current density flowing between the electrodes, in particular a manually actuatable member, and/or a device for automatically regulating the current density. The current density (A/cm²) may be regulated so as to ensure the efficacy of the treatment and/or to limit painful sensations. The regulation of the density of the current may be performed manually by the user, the latter being able to modify the magnitude of the current at will, or else when the warning facility signals to him that this is necessary, or automatically, by virtue of a system for slaving the current density.

The device may comprise a system for slaving the current density between the electrodes to the impedance measurement performed by the control system.

The warning facility may comprise one at least of a telltale light, of a sound warning facility, and of a vibratory warning facility, the warning facility preferably being triggered when the impedance measured by the control system lies in a predefined impedance range.

In one variant embodiment, the electric current between the electrodes is generated by a low-frequency current generator. In one variant embodiment, the electric current is not generated by a voltage generator.

The device may comprise an electronic timeout system configured to control the quantity of dispensed composition and/or the duration of the treatment.

### Control member

The device may comprise a composition dispensing control member, in particular a push-button (for example an ON/OFF button) or a rotary thumbwheel, and/or a device for regulating the composition flowrate. The control member is configured to be actuated manually by the user. The control member may be configured to allow the dispensing of a dose of composition, or as a variant a continuous stream of the composition. In one exemplary embodiment, the dispensing of composition continues as long as the control member is engaged, for example as long as the push-button is depressed.

The user may trigger the dispensing of composition when the quantity of composition present is insufficient to ensure the efficacy of the treatment. Regulation of the quantity of composition may be performed manually by the user, the latter being able to modify the quantity of composition dispensed at will, or else when the warning facility signals to him that this is necessary, or automatically, by virtue of a system for slaving the quantity of composition dispensed to a measurement performed on the keratinous material.

The device may comprise a system for slaving the quantity of composition exiting the reservoir to the impedance measurement performed by the control system. Slaving the dispensing of composition to the impedance of the skin makes it possible to ensure the presence of a sufficient quantity of cosmetic composition throughout the treatment. The quantity of composition may be adjusted automatically, as a function of the measurement performed.

The device may comprise a switch making it possible to place the device in automatic mode where the quantity of composition dispensed is slaved to the impedance measured by the control system or in semi-automatic mode where the warning facility advises the user on the need to modify the quantity of composition dispensed. By "semi-automatic mode" is meant that the warning facility may warn the user of the necessity to dispense composition manually. "Automatic mode" is understood to mean that the dispensing of composition is engaged automatically, as mentioned hereinabove, in case of necessity. In this case, the warning facility may not operate or as a variant may operate so as to signal to the user that composition will be dispensed.

### Reservoir

The member for dispensing the formula at the exit of the reservoir may comprise a pump. Advantageously, the dispensing member comprises an air pump so as to compress the air between the walls of the reservoir and its housing in the device. The walls of the reservoir are preferably flexible and the walls of the housing are preferably rigid.

More advantageously, the housing is airtight. The reservoir is compressed in a controlled manner by the air pump. This creates a certain pressure in the housing to regulate the flowrate of formula. A unidirectional valve at the end of the reservoir may make it possible to safeguard the formula from contact with the air.

The reservoir may have a variable internal volume and comprise at least one wall that is elastically deformable so as to reduce the internal volume, in particular two elastically deformable walls opposite one another.

The reservoir may be designed to be mounted in a removable manner on the device such that, when the reservoir has been emptied, it can be replaced with another or removed in order to be filled, when the reservoir has a filling orifice.

Advantageously, the reservoir has an elastically deformable outer wall.

Preferably, the reservoir comprises an exit orifice emerging through a duct in the diffuser.

Advantageously, the reservoir is formed as a single component. It is preferably molded in one piece, in particular from the same thermoplastic, for example LDPE, HDPE, a mixture of LDPE and HDPE, PP or a mixture of PE and PP in any proportion. The thickness of the wall of the reservoir is for example between 0.1 and 1 mm, and in the second zone between 0.3 and 0.8 mm.

Advantageously, the total internal volume of the reservoir is between 1 and 100 cm³, preferably between 20 and 50 cm³, in the rest configuration of the deformable zone. This volume is optimal for a few uses or a repeated treatment extending over a few weeks.

Preferably, the interior volume of the reservoir may shrink by a volume equal to between 10% and 50% of the interior volume of said reservoir in the rest configuration of the deformable zone. This variation in volume has the advantage of being visible and measurable.

The reservoir may be manufactured by injection blow molding or extrusion blow molding.

The reservoir may be removable. The reservoir may in particular be a single-use reservoir . It may or may not be mono dose.

### Composition

It is possible to use at least one cosmetic or dermatological composition with the device.

The composition(s) used may be in all forms, for example in the form of an aqueous solution, of an oil, of an emulsion, of a powder or of a gel. The composition(s) used may also be sprayed onto the skin.

When the composition(s) used is/are in the form of a gel, the latter can take on the shape of the electrode to which it is applied, as mentioned above.

The composition(s) may comprise an active principle.

Advantageously, the composition is chosen from among:
- a face care or body care composition, comprising in particular an active agent chosen from humectant or moisturizing active agents, anti-ageing active agents for example, depigmenting active agents, active agents that act on cutaneous microcirculation or seboregulating active agents,
- a composition for making up the face or body,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair, and
- a composition for the scalp, in particular an antidandruff composition, a composition for preventing hair loss or for promoting regrowth of the hair, an anti-seborrheic, anti-inflammatory, anti-irritation or soothing composition, a mark-preventing composition or a composition for stimulating or protecting the scalp.

The device may be used in diverse cosmetic or dermatological treatments, for example to combat wrinkles, herpes, acne or to redensify the skin or the hair.

### Description of the figures

The invention will be better understood on reading the detailed description which will follow, of nonlimiting examples of implementation of the invention, and on examining the appended drawing in which:
- Figures 1, 2, 4 and 5 are schematic and partial views, in perspective, of a device in accordance with the invention,
- Figure 3 is a schematic and partial view, in perspective, of the cap for closing the device of Figure 1,
- Figures 6a and 6b are schematic and partial views, in transverse cross-section, of the application member in its housing, respectively in the first position and in the second position,
- Figures 7a and 7b are schematic and partial views, in transverse cross-section, of the application member of a variant embodiment, in its housing, respectively in the first position and in the second position, and
- Figures 8a and 8b are schematic and partial views, in transverse cross-section, of the application member of another variant embodiment, in its housing, respectively in the first position and in the second position.

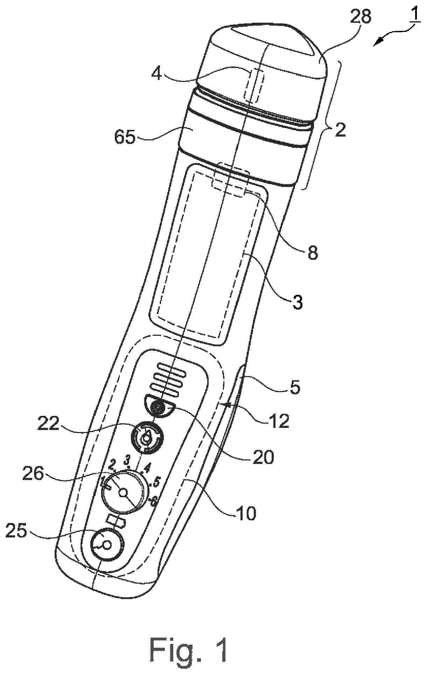

Represented in Figures 1 to 5 is a device 1 in accordance with the invention. It comprises a body 12 on which is mounted an end fitting 2 comprising six application members 6 each having a ball shape, and a composition reservoir 3 housed inside the body 12, and represented dashed. The end fitting 2 is fixed by snap-fastening on the body 12. It could be mounted in some other manner. In the example considered, the body 12 is made of thermoplastic material. As a variant, the latter may be made of any other material. Furthermore, the application members 6 are plastic balls. The application members 6 could have any other shape, being for example rollers.

The application members 6, six in number in the example described, are disposed as a triangle, thereby making it possible to facilitate the application and the treatment of zones that are difficult to access, for example the wings of the nose.

The device 1 comprises an electrode 4, visible dashed in Figure 1, the electrode 4 being housed inside the end fitting 2, as well as a counter-electrode 5 disposed on the body 12. The counter-electrode 5 is in contact with the hand of the user when the latter holds the device 1 in their hand, in such a way that the electrical circuit between the electrode 4 and the counter-electrode 5 closes, the current passing into the body of the user at the time of the treatment. The polarization of the electrode 4 and that of the counter-electrode 5 may be reversible (+ or -) according to the nature of the composition used.

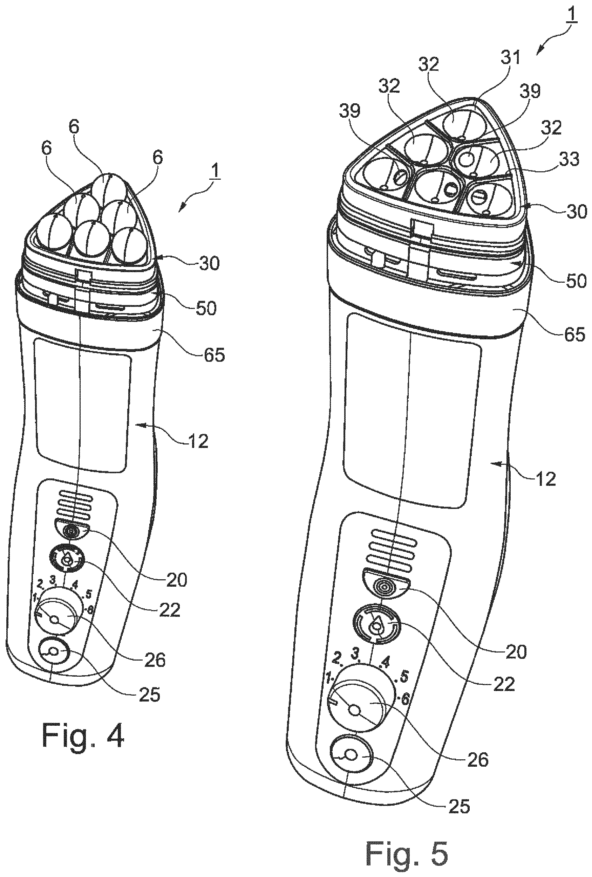

The electrode 4 is connected to a pole of an electrical supply system 10 housed in the body 12 and communicates with the composition when the latter passes into a distributor 30, visible in Figure 5. The composition thereafter passes over the application members 6 to arrive at the application surface, the skin in the example described, with a view to treatment. The counter electrode 5 is connected to the other pole of the electrical supply system 10, which is powered by a battery (cells or rechargeable battery).

To begin the treatment, the user turns on the device by means of a push-button 25. An electric current then begins to flow between the electrode 4 and the counter-electrode 5, when the apparatus is applied to the person's face for example, at the same time as the composition is dispensed by a member 8 for dispensing the composition contained in the reservoir, in particular a pump, automatically or by action of the user on a control member 22.

In a variant embodiment, the intensity of the current may be adjusted by the user by virtue of an adjustment member 26 for adjusting the current density between the electrodes, which is a graduated rotary button in the example of Figure 1.

If there is insufficient composition between the electrode 4 and the skin, the impedance of the skin increases. The device comprises a control system that is sensitive to the impedance of the skin and configured to detect such an impedance variation. The device also comprises a warning facility 20 making it possible to provide the user with a signal as a function of information originating from the control system. In the example described, the warning facility 20 provides a luminous signal. The user may then dispense an additional dose of composition by pressing the composition dispensing control member 22, which is a push-button in the example of Figure 1.

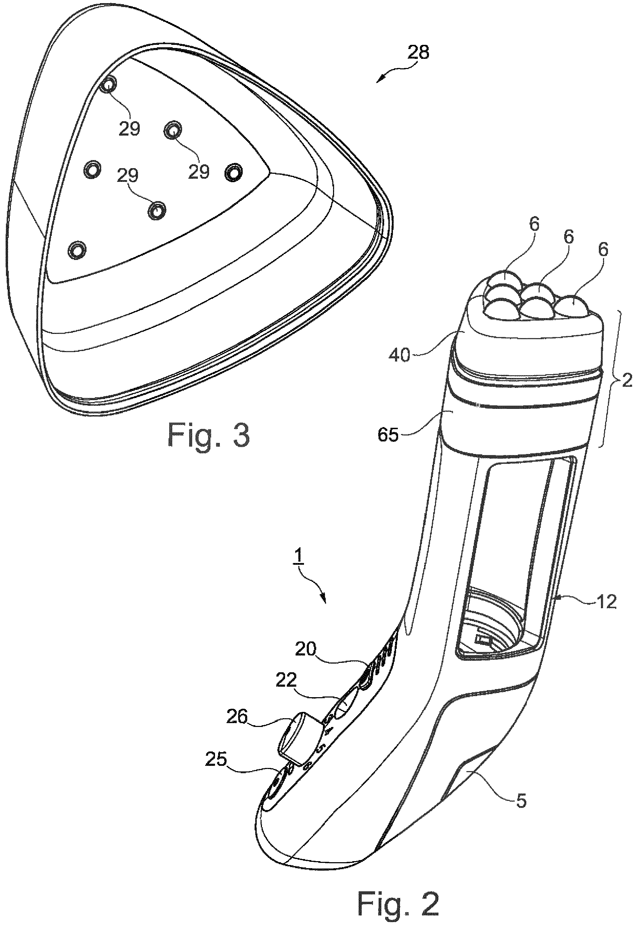

The structure of the end fitting 2 will now be described in greater detail with reference to Figures 2 to 6b.

The end fitting firstly comprises a closure cap 28, illustrated in isolation in Figure 3, which is configured to be fixed by snap-fastening onto the remainder of the device, and more particularly onto a cover 40 of the application members 6. The closure cap 28 comprises, in the bottom thereof, reliefs 29 for setting the application members 6. This closure cap is preferably not electrically conducting.

The cover 40 makes it possible to retain the application members 6 in place on the distributor 30. The cover 40 comprises openings through which the application members 6 protrude toward the exterior to allow application of the composition. The cover 40 is fixed on a diffuser 50, itself fixed on an upper ring 65 of the body 12. The diffuser 50 is directly in contact with the distributor 30, making it possible to feed the composition into the distributor.

The distributor 30, disposed under the cover 40, receives the plurality of application members 6, as illustrated in Figures 4 and 5. The distributor is electrically conducting, being formed from a thermoplastic material comprising an electrically conducting filler, in particular a carbon filler or a metallic filler. The distributor is electrically connected to the electrical supply system 10 so as to make it possible to ensure the transfer of the electric current from the electrical supply system 10 to the distributor and then to the composition. The distributor and the composition are the only elements in the end fitting to be electrically conducting.

The distributor 30 comprises a first face 31 on which are made housings 32 for the application members 6, in particular a housing 32 for each of the six application members 6. The first face 31 also comprises a rib 33 with several branches, separating the housings 32 from one another.

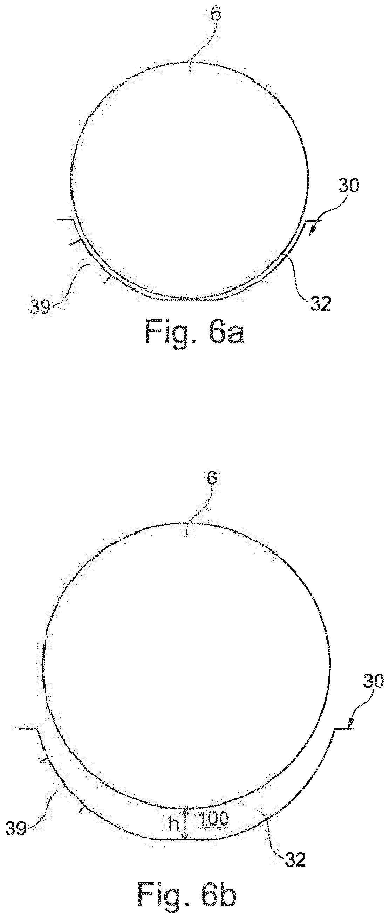

The application members are each movable in its housing 32 under the pressure of the composition between a first position illustrated in Figure 6a, which is the position of the application member when at rest in its housing, and a second position illustrated in Figure 6b, in which an intermediate storage chamber 100 for the composition is made between the bottom of the housing and the application member. The second position is the position of the application member which moves in its housing under the pressure of the composition.

The maximum volume of the intermediate chamber 100 is for example of the order of 20 µL. It corresponds to the clearance existing around the application member 6 when the latter is disposed in its housing 32.

A height *h* of the intermediate chamber 100 extends between the extreme positions of the application member.

The housing 32 is supplied with composition from the reservoir 3 through a supply orifice 39. The supply orifice may be disposed in the bottom of the housing, or as a variant be disposed elsewhere than in the bottom of the housing, as illustrated.

When a certain quantity of composition has been dispensed by the dispensing member from the reservoir, said quantity moves through the device to reach the intermediate chamber 100 where it may be stored before being dispensed onto the keratinous material by the application members. Under the pressure of the composition, each application member moves in its housing toward the second position, allowing the storage of the surplus composition. The application of composition by the application members may then continue, by using the surplus composition stored in the intermediate chamber, even if the dispensing of composition by the dispensing member from the reservoir is performed jerkily. Thus, the dispensing of the composition onto the keratinous material is smoothed.

In the example which has just been described, the application members 6 are retained in their housing by the cover 40, but it could be otherwise.

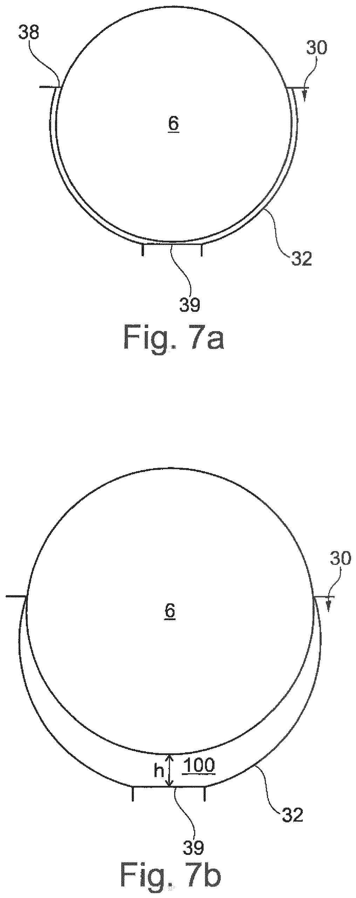

In the exemplary embodiment illustrated in Figures 7a and 7b, the housing is itself configured so as to retain the application member 6. The shape of the housing 32 is chosen so as to prevent the application member 6 from escaping from the latter. The housing 32 has in this example an opening 38 of smaller cross-section than a larger transverse cross-section of the application member 6. The opening 38 of the housing allows the application of the composition to the keratinous material by means of the application member which protrudes from the housing therethrough.

The housing 32 has a transverse cross-sectional shape, in a sectional plane perpendicular to the axis of the end fitting 2, that is larger than a semicircle, the application member being of circular shape in transverse cross-section.

In this exemplary embodiment, the bottom of the housing 32 is slightly flattened, just where the supply orifice is situated 39.

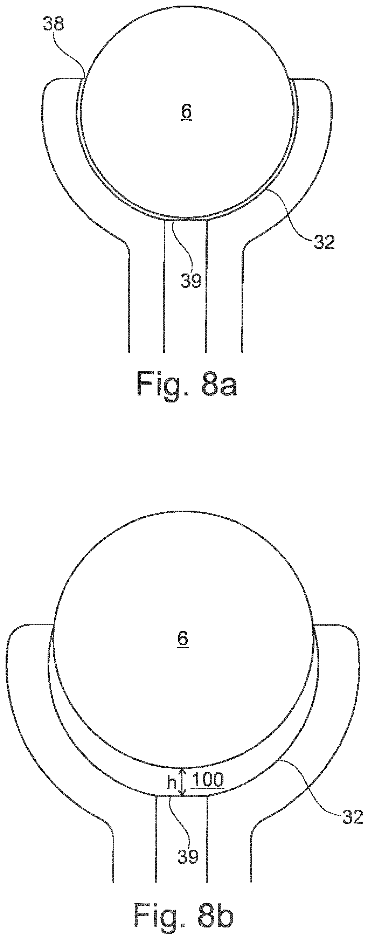

In the exemplary embodiment illustrated in Figures 8a and 8b, the application member has a cylinder shape, being for example a roller, and the housing has a shape slightly greater than a half-cylinder. The housing 32 is supplied with composition from the reservoir 3 through a supply orifice 39 disposed in the bottom of the housing, at the end of a vertical supply duct.

## Claims

1. A device (1) for treating human keratinous material, with the aid of an electric current, comprising at least:
- a reservoir (3) containing a composition (P), in particular cosmetic or dermatological, to be applied to the keratinous material,
- a dispensing member for dispensing the composition contained in the reservoir, in particular a pump,
- an end fitting (2) for the application of the composition contained in the reservoir (3), comprising at least one application member (6), in particular a plurality of application members (6), the application member (6) being received in a housing (32) supplied with composition from the reservoir (3) by the dispensing member, the application member being movable in its housing (32) under the pressure of the composition between a first position and a second position, at least one intermediate storage chamber (100) for the composition being delimited by the bottom of the housing and the application member, the volume of the intermediate chamber varying when the application member passes from its first position to its second position,
the device comprising an electrical supply system (10) for exposing the keratinous material to an electric treatment current in a zone of application of the composition.

2. The device as claimed in the preceding claim, comprising several application members (6), in particular between two and eight.

3. The device as claimed in any one of the preceding claims, wherein at least one application member is a ball and better still all the application members are balls.

4. The device as claimed in any one of the preceding claims, in which the maximum volume of the intermediate chamber (100) made between the bottom of the housing (32) and the application member (6) disposed in said housing (32) is between 5 and 100 microliters (µL), better between 10 and 50 µL.

5. The device as claimed in any one of the preceding claims, wherein the dispensing member is actuated manually.

6. The device as claimed in any one of the preceding claims, wherein the dispensing member is actuated automatically, for example by means of an electric motor.

7. The device as claimed in any one of the preceding claims, wherein the dispensing member is configured to dispense the composition jerkily in fits and starts.

8. The device as claimed in the preceding claim, wherein the dispensing member includes at least one motor, especially at least one non-servocontrolled geared motor.

9. The device as claimed in any one of the preceding claims, configured to allow the composition to be dispensed onto the keratinous material with a flow rate comprised between 0.5 and 20 µL/s and better still between 1 µL/s and 10 µL/s.

10. The device as claimed in any one of the preceding claims, in which the housing (32) is supplied with composition from the reservoir (3) through a supply orifice (39).

11. The device as claimed in any one of the preceding claims, in which the housing (32) has a shape configured so as to retain the application member (6) in the latter.

12. The device as claimed in any one of the preceding claims, in which the housing (32) has an opening of smaller cross-section than a larger transverse cross-section of the application member (6).

13. The device as claimed in the preceding claim, in which the housing has a transverse cross-sectional shape that is larger than a semicircle, the application member being of circular shape in transverse cross-section.

## Patentansprüche

1. Vorrichtung (1) zur Behandlung von menschlichem Keratinmaterial mit Hilfe von elektrischem Strom, umfassend zumindest:
- einen Behälter (3), der eine, insbesondere kosmetische oder dermatologische, Zusammensetzung (P) enthält, die auf das Keratinmaterial aufgetragen werden soll,
- ein Ausgabeelement zur Ausgabe der in dem Behälter enthaltenen Zusammensetzung, insbesondere eine Pumpe,
- ein Endanschlussstück (2) zum Auftragen der in dem Behälter (3) enthaltenen Zusammensetzung, umfassend zumindest ein Auftragelement (6), insbesondere mehrere Auftragelemente (6), wobei das Auftragelement (6) in einem Gehäuse (32) aufgenommen ist, das durch das Ausgabeelement mit Zusammensetzung aus dem Behälter (3) versorgt wird, wobei das Auftragelement in seinem Gehäuse (32) unter dem Druck der Zusammensetzung zwischen einer ersten Position und einer zweiten Position bewegbar ist, zumindest eine Zwischenspeicherkammer (100) für die Zusammensetzung, welche durch den Boden des Gehäuses und das Auftragelement begrenzt ist, wobei sich das Volumen der Zwischenkammer sich ändert, wenn das Auftragelement von seiner ersten Position in seine zweite Position übergeht,
wobei die Vorrichtung ein elektrisches Versorgungssystem (10) umfasst, um das Keratinmaterial in einer Zone der Auftragung der Zusammensetzung einem elektrischen Behandlungsstrom auszusetzen.

2. Vorrichtung nach dem vorhergehenden Anspruch, die mehrere, insbesondere zwischen zwei und acht, Auftragelemente (6) umfasst.

3. Vorrichtung nach einem der vorhergehenden Ansprüche, wobei mindestens ein Auftragelement eine Kugel ist und besser noch alle Auftragelemente Kugeln sind.

4. Vorrichtung nach einem der vorhergehenden Ansprüche, bei der das maximale Volumen der Zwischenkammer (100), die zwischen dem Boden des Gehäuses (32) und dem in diesem Gehäuse (32) angeordneten Auftragelement (6) gebildet wird, zwischen 5 und 100 Mikrolitern (µL), besser zwischen 10 und 50 µL, liegt.

5. Vorrichtung nach einem der vorhergehenden Ansprüche, wobei das Ausgabeelement manuell betätigt wird.

6. Vorrichtung nach einem der vorhergehenden Ansprüche, wobei das Ausgabeelement automatisch, z. B. mittels eines Elektromotors, betätigt wird.

7. Vorrichtung nach einem der vorhergehenden Ansprüche, wobei das Ausgabeelement so konfiguriert ist, dass es die Zusammensetzung ruckartig und stoßweise ausgibt.

8. Vorrichtung nach dem vorhergehenden Anspruch, wobei das Ausgabeelement mindestens einen Motor, insbesondere mindestens einen nichtservogesteuerten Getriebemotor, aufweist.

9. Vorrichtung nach einem der vorhergehenden Ansprüche, die so konfiguriert ist, dass die Zusammensetzung mit einer Fließgeschwindigkeit zwischen 0,5 und 20 µL/s und besser noch zwischen 1 µL/s und 10 µL/s auf das Keratinmaterial aufgetragen werden kann.

10. Vorrichtung nach einem der vorhergehenden Ansprüche, bei der das Gehäuse (32) durch eine Zuführungsöffnung (39) mit einer Zusammensetzung aus dem Vorratsbehälter (3) versorgt wird.

11. Vorrichtung nach einem der vorhergehenden Ansprüche, bei der das Gehäuse (32) eine Form aufweist, die so konfiguriert ist, dass das Auftragelement (6) in diesem gehalten wird.

12. Vorrichtung nach einem der vorhergehenden Ansprüche, bei der das Gehäuse (32) eine Öffnung mit kleinerem Querschnitt als ein größerer querverlaufender Querschnitt des Auftragelements (6) aufweist.

13. Vorrichtung nach dem vorhergehenden Anspruch, bei der das Gehäuse eine querverlaufende Querschnittsform aufweist, die größer als ein Halbkreis ist, wobei das Auftragelement im querverlaufenden Querschnitt eine kreisförmige Form aufweist.

## Revendications

1. Dispositif (1) de traitement des matières kératiniques humaines, notamment à l'aide d'un courant électrique, comportant au moins :
- un réservoir (3) contenant une composition (P), notamment cosmétique ou dermatologique, à appliquer sur les matières kératiniques,
- un organe de distribution de la composition contenue dans le réservoir, notamment une pompe,
- un embout (2) pour l'application de la composition contenue dans le réservoir (3), comportant au moins un organe d'application (6), notamment une pluralité d'organes d'application (6), l'organe d'application (6) étant reçu dans un logement (32) alimenté en composition depuis le réservoir (3) par l'organe de distribution, l'organe d'application étant mobile dans son logement (32) sous la pression de la composition entre une première position et une deuxième position, au moins une chambre intermédiaire (100) de stockage pour la composition étant délimitée par le fond du logement et l'organe d'application, le volume de la chambre intermédiaire variant lorsque l'organe d'application passe de sa première position à sa deuxième position,
le dispositif comprenant un système d'alimentation électrique (10) pour exposer les matières kératiniques à un courant électrique de traitement dans une zone d'application de la composition.

2. Dispositif selon la revendication précédente, comportant plusieurs organes d'application (6), notamment entre deux et huit.

3. Dispositif selon l'une quelconque des revendications précédentes, dans lequel au moins un organe d'application est une bille, mieux tous les organes d'application sont des billes.

4. Dispositif selon l'une quelconque des revendications précédentes, dans lequel le volume maximum de la chambre intermédiaire (100) ménagée entre le fond du logement (32) et l'organe d'application (6) disposé dans ledit logement (32) est compris entre 5 et 100 microlitres (µL), mieux entre 10 et 50 µL.

5. Dispositif selon l'une quelconque des revendications précédentes, dans lequel l'organe de distribution est actionné manuellement.

6. Dispositif selon l'une quelconque des revendications précédentes, dans lequel l'organe de distribution est actionné automatiquement, par exemple au moyen d'un moteur électrique.

7. Dispositif selon l'une quelconque des revendications précédentes, dans lequel l'organe de distribution est configuré pour distribuer la composition par à-coups.

8. Dispositif selon la revendication précédente, dans lequel l'organe de distribution comporte au moins un moteur, notamment au moins un moteur réducté sans asservissement.

9. Dispositif selon l'une quelconque des revendications précédentes, configuré pour permettre un débit de composition distribué sur les matières kératiniques compris entre 0,5 et 20 µL/s, mieux entre entre 1 µL/s et 10 µL/s.

10. Dispositif selon l'une quelconque des revendications précédentes, dans lequel le logement (32) est alimenté en composition depuis le réservoir (3) par un orifice d'alimentation (39).

11. Dispositif selon l'une quelconque des revendications précédentes, dans lequel le logement (32) a une forme configurée pour retenir l'organe d'application (6) dans celui-ci.

12. Dispositif selon l'une quelconque des revendications précédentes, dans lequel le logement (32) a une ouverture de section inférieure à une plus grande section transversale de l'organe d'application (6).

13. Dispositif selon la revendication précédente, dans lequel le logement a une forme en section transversale plus grande qu'un demi-cercle, l'organe d'application étant en section transversale de forme circulaire.
